# FASCICULE DE BREVET EUROPEEN

(11) **EP 3 743 025 B1**
(45) Date de publication et mention de la délivrance du brevet: **05.10.2022**
(21) Numéro de dépôt: 19701127.3
(22) Date de dépôt: 25.01.2019
(51) Int. Cl.: A61F 9/008, A61B 18/20, A61B 18/22, G02B 6/02

(54) **APPAREIL DE DECOUPE D'UN TISSU HUMAIN OU ANIMAL COMPORTANT UN COUPLEUR OPTIQUE**
VORRICHTUNG ZUM SCHNEIDEN EINES MENSCHLICHEN ODER TIERISCHEN GEWEBES MIT EINEM OPTISCHEN KOPPLER
APPARATUS FOR CUTTING A HUMAN OR ANIMAL TISSUE COMPRISING AN OPTICAL COUPLER

(30) Priorité: 25.01.2018 FR 1850577
(43) Date de publication de la demande: 02.12.2020
(73) Titulaire: Keranova, 42000 Saint Etienne (FR)
(72) Inventeur: NADOLNY, Sylvie, 42000 Saint-Etienne (FR); BAUBEAU, Emmanuel, 42000 SAINT ETIENNE (FR)
(74) Mandataire: Be IP
(86) Numéro de dépôt international: PCT/EP2019/051872
(87) Numéro de publication internationale: WO 2019/145484

(56) Documents cités:
- WO-A2-2008/055506
- FR-A1- 3 049 848
- US-A1- 2015 073 397
- RUSSELL P: "Photonic crystal fibers", SCIENCE,, vol. 299, 17 janvier 2003 (2003-01-17), pages 358-362, XP002286085, ISSN: 0036-8075, DOI: 10.1126/SCIENCE.1079280

## Description

### DOMAINE TECHNIQUE

La présente invention concerne le domaine technique du traitement de pathologies oculaires réalisé en utilisant un laser femtoseconde, et plus particulièrement celui de la chirurgie ophtalmologique pour notamment des applications de découpes de cornées, ou de cristallins.

L'invention concerne un dispositif de découpe d'un tissu humain ou animal, telle qu'une cornée, ou un cristallin, au moyen d'un laser femtoseconde.

Par laser femtoseconde, on entend une source lumineuse, apte à émettre un faisceau LASER sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

On a déjà proposé de réaliser des opérations chirurgicales de l'œil au moyen d'un laser femtoseconde, telles que des opérations de découpes de cornées ou de cristallins.

Le document FR 3 049 847 décrit un appareil de découpe d'un tissu humain ou animal, tel qu'une cornée, ou un cristallin. Cet appareil comporte :
- un laser femtoseconde pour générer un faisceau LASER,
- un système de mise en forme positionné sur la trajectoire dudit faisceau, pour moduler la phase du front d'onde du faisceau LASER de sorte à obtenir un faisceau LASER modulé en phase selon une consigne de modulation calculée pour répartir l'énergie du faisceau LASER en au moins deux points d'impact formant un motif dans son plan focal correspondant à un plan de découpe,
- un scanner optique de balayage disposé en aval du système de mise en forme pour déplacer le motif dans le plan de découpe en une pluralité de positions selon une direction de déplacement,
- un montage optique incluant des miroirs réfléchissants et des lentilles entre le système de mise en forme et le scanner optique pour la transmission du faisceau LASER modulé vers le scanner,
- un système optique de focalisation pour focaliser le faisceau LASER dans un plan de découpe.

L'utilisation d'un système de mise en forme permet de diminuer le temps de découpe du tissu biologique en générant plusieurs points d'impacts simultanément.

Par ailleurs, l'utilisation du système de mise en forme permet d'obtenir des points d'impact sensiblement égales (la forme, la position et le diamètre de chaque point étant contrôlés dynamiquement par un masque de phase calculé et affiché sur le système de mise en forme).

Ainsi, les bulles de gaz - générées par les points d'impact et - qui dilacèrent les tissus biologiques découpés sont de tailles sensiblement égales.

Ceci permet d'améliorer la qualité du résultat obtenu, avec un plan de découpe homogène, dans lequel les ponts tissulaires résiduels (entre points d'impact adjacents) ont tous sensiblement la même taille. Cette homogénéité dans la taille des ponts tissulaires permet une dissection par le praticien d'une qualité acceptable au regard de l'importance de la qualité de l'état de surface du tissu découpé lorsqu'il s'agit par exemple d'une cornée.

Toutefois, pour faciliter l'opération de dissection par le praticien, il est préférable de réduire la taille des ponts tissulaires résiduels entre points d'impact adjacents.

Cette taille des ponts tissulaires dépendant de l'homogénéité des différents points d'impact, un but de la présente invention est de proposer une solution technique permettant d'améliorer l'homogénéité de la répartition d'énergie entre les différents points d'impact générés simultanément grâce au système de mise en forme.

Un autre but de la présente invention est de proposer une solution technique permettant d'améliorer l'appareil décrit dans FR 3 049 847 afin de réduire la taille des ponts tissulaires résiduels entre points d'impact adjacents.

Un autre but encore de la présente invention est d'améliorer la sécurité de l'appareil décrit dans FR 3 049 847 en y incorporant un organe de sécurité permettant d'interrompre la transmission du faisceau laser jusqu'au tissu à traiter si ledit faisceau laser se désaxe (par exemple en cas de choc sur l'appareil).

### EXPOSE DE L'INVENTION

A cet effet, l'invention propose un Appareil de découpe d'un tissu humain ou animal, tel qu'une cornée, ou un cristallin, ledit appareil incluant
- un laser femtoseconde pour émettre un faisceau LASER initial sous la forme d'impulsions,
- un système de mise en forme - tel qu'un modulateur spatial de lumière (SLM) - positionné en aval du laser femtoseconde, pour transformer le faisceau LASER initial en faisceau LASER modulé en phase, le système de mise en forme étant apte à moduler la phase du front d'onde du faisceau LASER initial selon une consigne de modulation calculée pour répartir l'énergie du faisceau LASER en au moins deux points d'impact formant un motif dans un plan de focalisation,
- un scanner optique de balayage, positionné en aval du système de mise en forme, pour déplacer le motif le long d'un chemin de déplacement prédéfini dans le plan de focalisation,
- un système optique de focalisation, positionné en aval du scanner optique de balayage, pour déplacer le plan de focalisation du faisceau LASER modulé dans un plan de découpe du tissu désiré,
- une unité de commande permettant de piloter le système de mise en forme, le scanner optique de balayage et le système optique de focalisation,
remarquable en ce que l'appareil comprend en outre un coupleur optique entre le laser femtoseconde et le système de mise en forme, le coupleur optique incluant une fibre optique à cristal photonique pour le filtrage du faisceau LASER issu du laser femtoseconde.

On entend, dans le cadre de la présente invention, par « *point d'impact »* une zone du faisceau LASER comprise dans son plan focal dans laquelle l'intensité dudit faisceau LASER est suffisante pour générer une bulle de gaz dans un tissu.

On entend, dans le cadre de la présente invention, par *« points d'impact adjacents »,* deux points d'impact disposés en regard l'un de l'autre et non séparés par un autre point d'impact. On entend par « *points d'impact voisins* » deux points d'un groupe de points adjacents entre lesquels la distance est minimale.

On entend, dans le cadre de la présente invention, par « *motif »* une pluralité de points d'impact LASER générés simultanément dans un plan de focalisation d'un faisceau LASER mis en forme - c'est-à-dire modulé en phase pour répartir son énergie en plusieurs spots distincts dans le plan de focalisation correspondant au plan de découpe du dispositif.

Ainsi, l'invention permet de modifier le profil d'intensité du faisceau LASER dans le plan de la découpe, d'une manière à pouvoir améliorer la qualité ou bien la vitesse de la découpe en fonction du profil choisi. Cette modification de profil d'intensité est obtenue par modulation de la phase du faisceau LASER

La modulation optique de phase est réalisée au moyen d'un masque de phase. L'énergie du faisceau LASER incident est conservée après modulation, et la mise en forme du faisceau est réalisée en agissant sur son front d'onde. La phase d'une onde électromagnétique représente la situation instantanée de l'amplitude d'une onde électromagnétique. La phase dépend aussi bien du temps que de l'espace. Dans le cas de la mise en forme spatiale d'un faisceau LASER, seules les variations dans l'espace de la phase sont considérées.

Le front d'onde est défini comme la surface des points d'un faisceau possédant une phase équivalente (i.e. la surface constituée des points dont les temps de parcours depuis la source ayant émis le faisceau sont égaux). La modification de la phase spatiale d'un faisceau passe donc par la modification de son front d'onde.

Cette technique permet de réaliser l'opération de découpe d'une manière plus rapide et plus efficace car elle met en œuvre plusieurs spots LASER réalisant chacun une découpe et selon un profil contrôlé.

Le fait de positionner le coupleur optique incluant la fibre optique à cristal photonique entre le laser femtoseconde et le système de mise en forme (plutôt qu'entre le système de mise en forme et le scanner optique de balayage) permet d'éviter toute perturbation dans la mise en forme du faisceau laser réalisée par le système de mise en forme. En effet, l'introduction d'un coupleur optique incluant une fibre à cristal photonique entre le système de mise en forme et le scanner optique de balayage induirait un filtrage du faisceau laser modulé (issu du système de mise en forme) tendant à dégrader sa mise en forme, et à diminuer sa puissance.

La fibre est une fibre à cristal photonique à cœur creux, ladite fibre incluant un cœur creux, et au moins une gaine entourant le cœur creux ;
- le coupleur optique comprend en outre :
   - une première cellule de liaison pour le raccordement du coupleur optique au système de mise en forme d'une part, et
   - une deuxième cellule de liaison pour le raccordement du coupleur optique au scanner optique de balayage ;
- chaque cellule de liaison peut être montée de manière étanche à une extrémité respective de la fibre à cristal photonique ;
- chaque cellule de liaison peut comprendre :
   - une enveloppe externe,
   - un canal de transmission logé dans l'enveloppe, le canal de transmission permettant le passage du faisceau LASER à l'intérieur de l'enveloppe,
   - un hublot transparent au rayonnement LASER à une extrémité du canal de transmission, le hublot étant destiné à venir en regard du laser femtoseconde ou du système de mise en forme ;
- l'appareil comprend au moins une pompe à vide, chaque cellule de liaison comprenant au moins une borne de connexion débouchant vers l'extérieur de l'enveloppe et étant destinée à être raccordée à la pompe à vide ;
- l'unité de commande peut comprendre des moyens aptes à piloter l'activation de la pompe à vide pour aspirer les gaz contenus dans le cœur creux de la fibre optique à cristal photonique.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique d'un montage incluant l'appareil de découpe selon l'invention ;
- la figure 2 illustre une répartition d'intensité d'un faisceau LASER dans son plan focal ;
- la figure 3 illustre un exemple de coupleur optique de l'appareil de découpe illustré à la figure 1 ;
- la figure 4 illustre un chemin de déplacement d'un motif de découpe ;
- la figure 5 illustre des plans de découpe d'un volume de tissu à détruire ;
- la figure 6 illustre un appareil de thérapie incluant un bras articulé.

### EXPOSE DETAILLE DE L'INVENTION

L'invention concerne un appareil de découpe d'un tissu humain ou animal au moyen d'un laser femtoseconde. Dans la suite de la description, l'invention sera décrite, à titre d'exemple, pour la découpe d'une cornée d'un œil humain ou animal.

### 1. Appareil de découpe

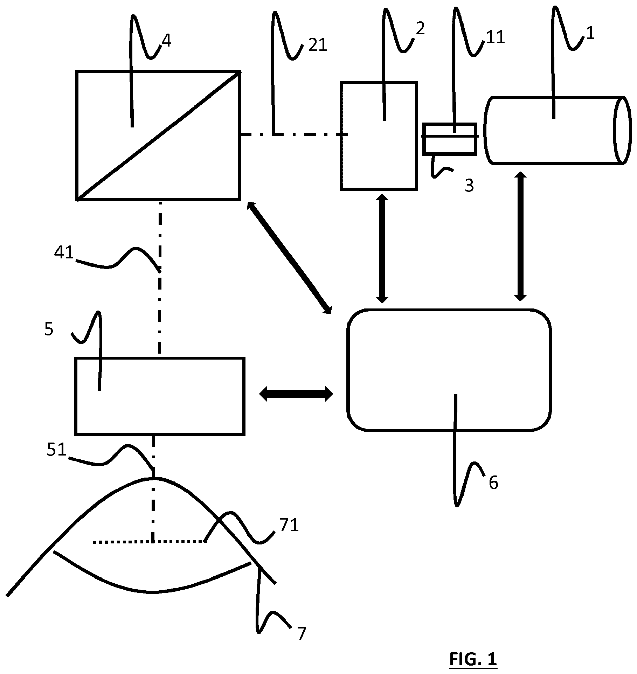

En référence à la figure 1, on a illustré un mode de réalisation de l'appareil de découpe selon l'invention. Celui-ci peut être disposé en amont d'une cible à traiter 7. La cible 7 est par exemple un tissu humain ou animal à découper tel qu'une cornée ou un cristallin.

L'appareil de découpe comprend :
- un laser femtoseconde 1,
- un système de mise en forme 2 positionné en aval du laser femtoseconde 1,
- un coupleur optique 3 entre le laser femtoseconde 1 et le système de mise en forme 2,
- un scanner optique de balayage 4 en aval du système de mise en forme 2,
- un système optique de focalisation 5 en aval du scanner optique de balayage 4,
- une unité de commande 6 permettant de piloter le laser femtoseconde 1, le système de mise en forme 2, le scanner optique de balayage 4 et le système optique de focalisation 5.

Le laser femtoseconde 1 est apte à émettre un faisceau LASER initial sous la forme d'impulsions. A titre d'exemple, le laser 1 émet une lumière de 1030 nm de longueur d'onde, sous la forme d'impulsions de 400 femtosecondes. Le laser 1 possède une puissance comprise entre 2 et 20W et de préférence de l'ordre de 8W et une fréquence comprise entre 100 et 500 kHz.

Le coupleur optique 3 permet de transmettre le faisceau LASER 11 issu du laser femtoseconde 1 vers le système de mise en forme 2.

Le système de mise en forme 2 s'étend sur la trajectoire du faisceau LASER initial 11 issu du laser femtoseconde 1. Il permet de transformer le faisceau LASER initial 11 en un faisceau LASER modulé 21. Plus précisément, le système de mise en forme 2 permet de moduler la phase du faisceau LASER 11 pour répartir l'énergie du faisceau LASER en une pluralité de points d'impact dans son plan focal, cette pluralité de points d'impact définissant un motif 8.

Le scanner optique de balayage 4 permet d'orienter le faisceau LASER modulé 21 pour déplacer le motif 8 le long d'un chemin de déplacement prédéfini par l'utilisateur dans un plan de focalisation 71.

Le système optique de focalisation 5 permet de déplacer le plan de focalisation 71 - correspondant au plan de découpe - du faisceau LASER dévié 41 issu du scanner optique de balayage 4.

Ainsi :
- le coupleur optique 3 permet de propager le faisceau LASER 11 entre le laser femtoseconde et le système de mise en forme 2,
- le système de mise en forme 2 permet de générer simultanément plusieurs points d'impact 81 définissant un motif 8,
- le scanner optique de balayage 4 permet de déplacer ce motif 8 dans le plan de focalisation 71, et
- le système optique de focalisation 5 permet de déplacer le plan de focalisation 71 en profondeur de sorte à générer des découpes dans des plans successifs définissant un volume.

Les différents éléments constituant l'appareil de découpe vont maintenant être décrits plus en détails en référence aux figures.

### 2. Eléments de l'appareil de découpe

### 2.1. Système de mise en forme

Le système de mise en forme spatiale 2 permet de faire varier la surface d'onde du faisceau LASER initial 11 pour obtenir des points d'impact 8 séparés les uns des autres dans le plan de focalisation 71.

Plus précisément, le système de mise en forme 2 permet de moduler la phase du faisceau LASER initial 11 issu du laser femtoseconde 1 pour former des pics d'intensité dans le plan focalisation 71, chaque pic d'intensité produisant un point d'impact respectif dans le plan focal correspondant au plan de découpe. Le système de mise en forme 2 est, selon le mode de réalisation illustré, un modulateur spatial de lumière à cristaux liquides, connu sous le sigle SLM, de l'acronyme anglais « *Spatial Light Modulator ».*

Le SLM permet de moduler la répartition finale d'énergie du faisceau LASER, notamment dans le plan de focalisation 71 correspondant au plan de découpe du tissu 7. Plus précisément, le SLM est adapté pour modifier le profil spatial du front d'onde du faisceau LASER primaire 11 issu du laser femtoseconde 1 pour distribuer l'énergie du faisceau LASER en différents spots de focalisation dans le plan de focalisation 71.

La modulation en phase du front d'onde peut être vue comme un phénomène d'interférences en deux dimensions. Chaque portion du faisceau LASER initial 11 issu de la source 1 est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact 81 n'a lieu que dans un seul plan (i.e. le plan de focalisation 71) et pas tout au long du chemin de propagation du faisceau LASER modulé. Ainsi, l'observation du faisceau LASER modulé avant ou après le plan de focalisation ne permet pas d'identifier une redistribution de l'énergie en une pluralité de points d'impact 81 distincts, du fait de ce phénomène qu'on peut assimiler à des interférences constructives (qui n'ont lieu que dans un plan et pas tout au long de la propagation comme dans le cas de la séparation d'un faisceau LASER initial en une pluralité de faisceaux LASER secondaires).

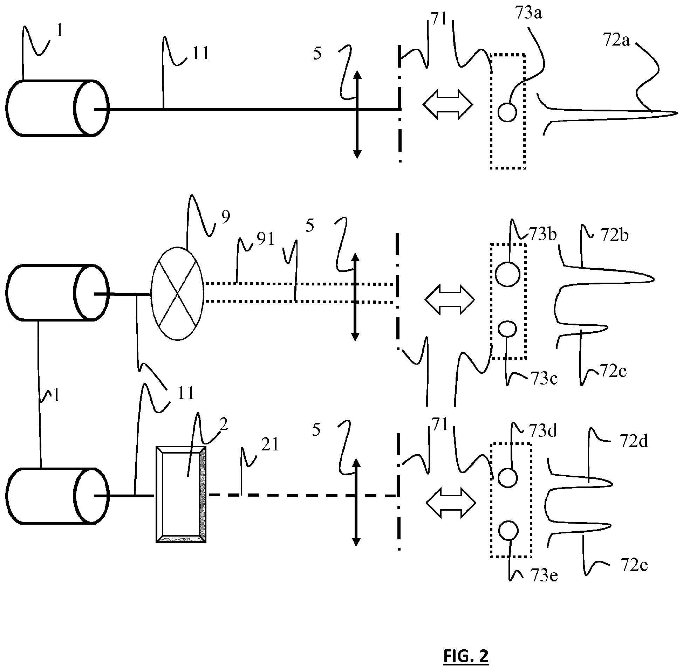

Pour mieux comprendre ce phénomène de modulation de phase du front d'onde, on a illustré schématiquement à la figure 2 des profils d'intensité 72a-72e obtenus pour trois exemples de montages optiques distincts. Comme représenté à la figure 2, un faisceau LASER initial 11 émis par une source laser 1 produit un pic d'intensité 72a de forme gaussienne en un point d'impact 73a dans un plan de focalisation 71. L'insertion d'un séparateur de faisceau 9 entre la source 1 et le plan de focalisation 71 induit la génération d'une pluralité de faisceau LASER secondaires 91, chaque faisceau LASER secondaire 91 produisant un point d'impact 73b, 73c respectif dans le plan de focalisation 71 des faisceaux LASER secondaires 91. Enfin, l'insertion entre la source 1 et le plan de focalisation 71 d'un SLM 2 programmé à l'aide d'un masque de phase formant consigne de modulation induit la modulation de la phase du front d'onde du faisceau LASER initial 11 issu de la source 1. Le faisceau LASER 21 dont la phase du front d'onde a été modulée permet d'induire la production de plusieurs pics d'intensité 73d, 73e séparés spatialement dans le plan de focalisation 71, chaque pic 72d, 72e correspondant à un point d'impact 73d, 73e respectif réalisant une découpe. La technique de modulation de la phase du front d'onde permet de générer dans le tissu cible plusieurs bulles de gaz simultanées sans démultiplication du faisceau LASER initial 11 produit par le laser femtoseconde 1.

Le SLM est un dispositif constitué d'une couche de cristaux liquides à orientation contrôlée permettant de façonner d'une manière dynamique le front d'onde, et donc la phase du faisceau LASER. La couche de cristaux liquides d'un SLM est organisée comme une grille (ou matrice) de pixels. L'épaisseur optique de chaque pixel est contrôlée électriquement par orientation des molécules de cristal liquide appartenant à la surface correspondant au pixel. Le SLM exploite le principe d'anisotropie des cristaux liquides, c'est-à-dire la modification de l'indice des cristaux liquides, en fonction de leur orientation spatiale. L'orientation des cristaux liquides peut être réalisée à l'aide d'un champ électrique. Ainsi, la modification de l'indice des cristaux liquides modifie le front d'onde du faisceau LASER.

D'une manière connue, le SLM met en œuvre un masque de phase, c'est-à-dire une carte déterminant comment la phase du faisceau doit être modifiée pour obtenir une répartition d'amplitude donnée dans son plan de focalisation 71. Le masque de phase est une image bidimensionnelle dont chaque point est associé à un pixel respectif du SLM. Ce masque de phase permet de piloter l'indice de chaque cristal liquide du SLM en convertissant la valeur associée à chaque point du masque - représentée en niveaux de gris compris entre 0 et 255 (donc du noir au blanc) - en une valeur de commande - représentée en une phase comprise entre 0 et 2π. Ainsi, le masque de phase est une consigne de modulation affichée sur le SLM pour entraîner en réflexion un déphasage spatial inégal du faisceau LASER illuminant le SLM. Bien entendu, l'homme du métier appréciera que la plage de niveau de gris peut varier en fonction du modèle de SLM utilisé. Par exemple dans certains cas, la plage de niveau de gris peut être comprise entre 0 et 220. Le masque de phase est généralement calculé par un algorithme itératif basé sur la transformée de Fourier, ou sur divers algorithmes d'optimisation, tels que des algorithmes génétiques, ou le recuit simulé. Différents masques de phase peuvent être appliqués aux SLM en fonction du nombre et de la position des points d'impact souhaités dans le plan focal du faisceau LASER. Dans tous les cas, l'homme du métier sait calculer une valeur en chaque point du masque de phase pour distribuer l'énergie du faisceau LASER en différents spots de focalisation dans le plan de focal.

Le SLM permet donc, à partir d'un faisceau LASER gaussien générant un unique point d'impact, et au moyen du masque de phase, de répartir son énergie par modulation de phase de sorte à générer simultanément plusieurs points d'impact dans son plan de focalisation à partir d'un unique faisceau LASER mis en forme par modulation de phase (un seul faisceau en amont et en aval du SLM).

En plus d'une diminution du temps de découpe de la cornée, la technique de modulation de la phase du faisceau LASER permet d'autres améliorations, telles qu'une meilleure qualité de surface après découpe ou une diminution de la mortalité endothéliale. Les différents points d'impact du motif peuvent, par exemple, être régulièrement espacés sur les deux dimensions du plan focal du faisceau LASER, de manière à former un quadrillage de spots LASER

Ainsi, le système de mise en forme 2 permet de réaliser une opération de découpe chirurgicale d'une manière rapide et efficace. Le SLM permet de façonner d'une manière dynamique le front d'onde du faisceau LASER puisqu'il est paramétrable numériquement. Cette modulation permet la mise en forme du faisceau LASER d'une manière dynamique et reconfigurable.

Le SLM peut être configuré pour mettre en forme le front d'onde du faisceau LASER de toute autre manière. Par exemple, chaque point d'impact peut présenter une forme géométrique quelconque, autre que circulaire (par exemple en ellipse, etc.). Ceci peut présenter certains avantages en fonction de l'application considérée, comme une augmentation de la vitesse et/ou de la qualité de la découpe.

### 2.2. Coupleur optique

Le coupleur optique 3 permet la transmission du faisceau LASER 11 entre le laser femtoseconde 1 et le système de mise en forme 2.

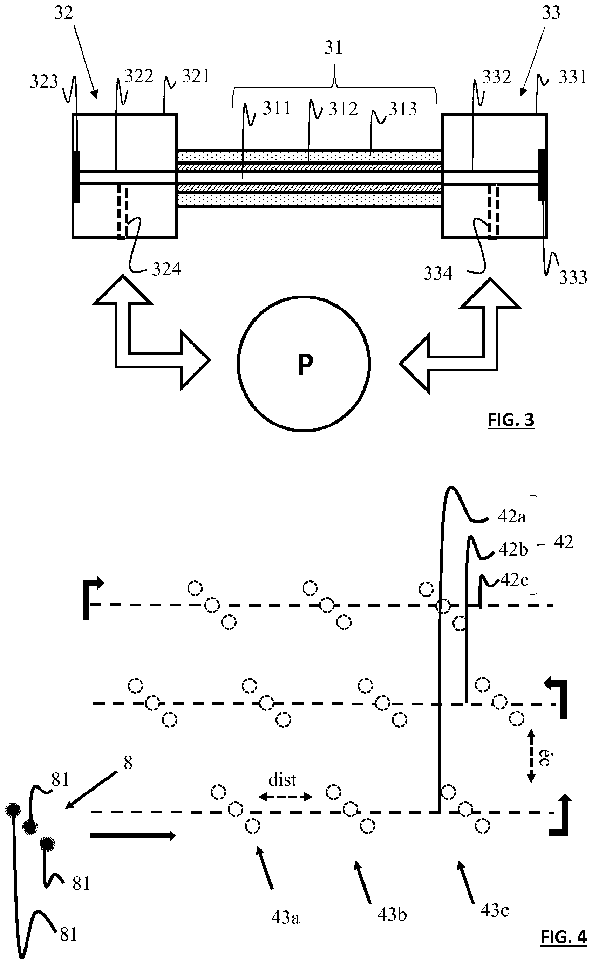

En référence à la figure 3, le coupleur optique 3 comprend une fibre optique 31. Ceci permet au coupleur optique 3 de constituer un « *fusible optique ».* En effet, si la direction du faisceau LASER 11 (i.e. son point de visé) est modifiée brusquement - par exemple en cas de choc sur le dispositif de découpe - alors le faisceau LASER 11 ne pénètre plus dans la fibre, ce qui limite les risques d'erreur lors du traitement d'un patient. Ceci n'est pas possible avec un montage optique incluant des miroirs réfléchissants et des lentilles pour la transmission du faisceau LASER issu du laser femtoseconde.

La fibre optique 31 est une fibre à cristal photonique.

Une fibre à cristal photonique, ou *« PCF »* (sigle de l'expression anglo-saxonne *« Photonic-Crystal Fiber* ») sont des guides d'onde formés d'un réseau périodique en deux dimensions d'inclusions qui s'étendent sur toute la longueur de la fibre. La transmission d'un faisceau LASER à travers une telle fibre est basée sur les propriétés des cristaux photoniques. Grâce à leurs structures, ces fibres assurent le confinement des ondes électromagnétiques dans le cœur de la fibre. Ces fibres à cristaux photoniques offrent une grande variété de possibilités pour le guidage en ajustant leurs paramètres optogéométriques comme par exemple le diamètre des inclusions, la répartition des inclusions, la périodicité (pas entre deux inclusions), le nombre de couches, l'indice des matériaux utilisés.

La fibre optique 31 est une fibre à cristal photonique à cœur creux. Une fibre à cristal photonique à cœur creux est une fibre optique qui guide la lumière essentiellement à l'intérieur d'une région creuse (le cœur de la fibre), de sorte que seule une partie mineure de la puissance optique se propage dans le matériau de fibre solide (typiquement de la silice). Selon le mécanisme physique standard pour guider la lumière dans une fibre, ceci ne devrait pas être possible : normalement, l'indice de réfraction du cœur de fibre doit être plus élevé que celui du matériau de gainage environnant, et il n'y a aucun moyen d'obtenir un indice de réfraction de verre au-dessous de celui de l'air ou du vide, au moins dans la région optique. Cependant, un mécanisme de guidage différent peut être utilisé, basé sur une bande interdite photonique, comme cela peut être réalisé dans une fibre à cristal photonique. De telles fibres sont également appelées fibres à bande interdite photonique. Les attraits des fibres à cristal photonique à cœur creux sont principalement que le guidage primaire dans la région creuse minimise les effets non linéaires du faisceau LASER 11 et permet un seuil de dommage élevé.

A titre d'exemple, le document FR 3 006 774 décrit un guide d'onde sous forme d'une fibre à cristaux photoniques à cœur creux comprenant une gaine, l'absence de capillaire en partie centrale formant le cœur creux. L'utilisation d'une fibre à cristal photonique à cœur creux permet de filtrer le faisceau LASER 11 issu du laser femtoseconde 1 afin de faciliter sa mise en forme par le système de mise en forme 2. Plus précisément, l'utilisation d'une fibre à cristal photonique à cœur creux permet de limiter la divergence du faisceau LASER 11 (i.e. profil étalé) en améliorant sa directivité (ce qui rend le faisceau LASER 11 plus propre en limitant l'étalement de son profil). En effet, une fibre à cristal photonique à cœur creux permet de confiner la lumière plus efficacement qu'une fibre classique à cœur plein. La fibre à cristal photonique à cœur creux comprend :
- un cœur creux 311,
- une gaine interne 312 à base de silice entourant le cœur creux, la gaine interne ayant un indice de réfraction n1<nc, où nc est l'indice de réfraction efficace du cœur creux,
- une gaine externe 313 entourant la gaine interne 312.

La région creuse 311 de la fibre à cristal photonique à cœur creux est mise sous vide pour limiter les pertes de propagation du faisceau LASER 11 issus du laser femtoseconde 1. En variante un gaz peut être injecté dans la région creuse pour exploiter l'intensité optique élevée dans la fibre - par exemple pour une génération d'harmonique élevée du faisceau LASER 11 issu du laser femtoseconde 1. A cet effet, le coupleur optique 3 comprend des première et deuxième cellules de liaison 32, 33 montées de manière étanche à chaque extrémité de la fibre à cristal photonique à cœur creux.

Chaque cellule de liaison 32, 33 comprend :
- une enveloppe externe 321, 331,
- un canal de transmission 322, 332 logé dans l'enveloppe 321, 331, le canal de transmission 322, 332 permettant le passage du faisceau LASER 11 à l'intérieur de l'enveloppe 321, 331,
- un hublot 323, 333 transparent au rayonnement LASER à une extrémité du canal de transmission 322, 332 pour l'entrée (ou la sortie) du faisceau LASER 11,
- un connecteur (non représenté) à l'autre extrémité du canal de transmission, le connecteur étant raccordé de manière étanche à une extrémité de la fibre optique 31,
- une borne de connexion 324, 334 débouchant vers l'extérieur de l'enveloppe 321, 331 et étant destinée à être raccordée à une pompe à vide P.

L'activation de la pompe à vide P permet de mettre le cœur creux 311 de la fibre optique 31 sous vide par un pompage au niveau des cellules de liaisons 32, 33 situées aux deux extrémités de la fibre optique 31. Le fait de réaliser un pompage sous vide à chaque extrémité de la fibre optique 31 permet de faciliter la mise sous vide du cœur creux sur toute la longueur de la fibre optique 31.

### 2.3. Scanner optique de balayage

Le scanner optique de balayage 4 permet de dévier le faisceau LASER modulé en phase 21 de sorte à déplacer le motif 8 en une pluralité de positions 43a-43c dans le plan de focalisation 71 correspondant au plan de découpe.

Le scanner optique de balayage 4 comprend :
- un orifice d'entrée raccordé au coupleur optique 3 pour recevoir le faisceau LASER modulé en phase 21 issu de l'unité de mise en forme 2,
- un (ou plusieurs) miroir(s) optique(s) pivotant autour d'au moins deux axes pour dévier le faisceau LASER modulé en phase 21, et
- un orifice de sortie pour envoyer le faisceau LASER modulé dévié 41 vers le système optique de focalisation 5.

Le scanner optique 4 utilisé est par exemple une tête de balayage IntelliScan III de la société SCANLAB AG. Les orifices d'entrée et de sortie d'un tel scanner optique 4 présentent un diamètre de l'ordre de 10 à 20 millimètres, et les vitesses de balayage atteignables sont de l'ordre de 1m/s à 10 m/s suivant la longueur focale de l'optique utilisée.

Le (ou les) miroir(s) est (sont) connecté(s) à un (ou des) moteur(s) pour permettre leur pivotement. Ce(s) moteur(s) pour le pivotement du (ou des) miroir(s) est (sont) avantageusement piloté(s) par l'unité de l'unité de commande 6 qui sera décrite plus en détails dans la suite.

L'unité de commande 6 est programmée pour piloter le scanner optique de balayage 4 de sorte à déplacer le motif 8 le long d'un chemin de déplacement 42 contenu dans le plan de focalisation 71. Dans certains modes de réalisation, le chemin de déplacement 42 comprend une pluralité de segments de découpe 42a-42c. Le chemin de déplacement 42 peut avantageusement présenter une forme de créneau, ou en spirale, etc.

Le balayage du faisceau a une grande importance pour le résultat de la découpe obtenu. En effet, la vitesse de balayage utilisée, ainsi que le pas du balayage, sont des paramètres influençant la qualité de la découpe.

L'utilisation d'un coupleur optique incluant une fibre optique 31 de type à cristal à corps creux (plutôt qu'un montage optique composé de miroirs afin de guider le faisceau LASER 11) permet, lors de l'utilisation d'une mise en forme multipoints 81, d'améliorer l'homogénéité de la répartition d'énergie entre les points dans le cas limite de points d'impact très rapprochés (espacement centre à centre entre deux points mis en forme inférieur au diamètre d'un point).

Dans un mode de réalisation, l'appareil de découpe comprend en outre un prisme de Dove. Celui-ci est avantageusement positionné entre le couleur optique 3 et le scanner optique de balayage 4. Le prisme de Dove permet de mettre en œuvre une rotation du motif 8 qui peut être utile dans certaines applications ou pour limiter la taille de la zone d'amorçage de chaque segment de découpe 42a-42c.

Avantageusement, l'unité de commande 6 peut être programmée pour activer le laser femtoseconde 1 lorsque la vitesse de balayage du scanner optique 4 est supérieure à une valeur seuil. Ceci permet de synchroniser l'émission du faisceau LASER 11 avec le balayage du scanner optique de balayage 4. Plus précisément, l'unité de commande 6 active le laser femtoseconde 1 lorsque la vitesse de pivotement du (ou des) miroir(s) du scanner optique 4 est constante. Ceci permet d'améliorer la qualité de découpe par la réalisation d'un surfaçage homogène du plan de découpe.

### 2.4. Système optique de focalisation

Le système optique de focalisation 5 permet de déplacer le plan de focalisation 71 du faisceau LASER modulé et dévié 41 dans un plan de découpe du tissu 7 désiré par l'utilisateur.

Le système optique de focalisation 5 comprend :
- un orifice d'entrée pour recevoir le faisceau LASER modulé en phase et dévié issu du scanner optique de balayage 4,
- une (ou plusieurs) lentille(s) motorisée(s) pour permettre son (leur) déplacement en translation le long du chemin optique du faisceau LASER modulé en phase et dévié, et
- un orifice de sortie pour envoyer le faisceau LASER focalisé vers le tissu à traiter.

La (ou les) lentilles utilisées avec le système optique de focalisation 5 peuvent être des lentilles à champ plan. Les lentilles à champ plan permettent d'obtenir un plan de focalisation sur tout le champ XY, contrairement aux lentilles standard pour lesquelles il est concave. Cela permet de garantir une taille de faisceau focalisé constante sur tout le champ.

L'unité de commande 6 est programmée pour piloter le déplacement de la (ou des) lentille(s) du système optique de focalisation 5 le long d'un chemin optique du faisceau LASER de sorte à déplacer le plan de focalisation 71 en au moins trois plans de découpe respectifs 72a-72e de sorte à former un empilement de plans de découpe du tissu 7. Ceci permet d'effectuer une découpe dans un volume 74, par exemple dans le cadre d'une chirurgie réfractive.

L'unité de commande 6 est apte à piloter le déplacement du système optique de focalisation 5 pour déplacer le plan de focalisation 71 entre une première position extrême 72a et une deuxième position extrême 72e, dans cet ordre. Avantageusement, la deuxième position extrême 72e est plus proche du laser femtoseconde 1 que la première position extrême 72a.

Ainsi, les plans de découpe 72a-72e sont formés en commençant par le plan de découpe le plus profond 72a dans le tissu et en empilant les plans de découpe successifs jusqu'au plan de découpe le plus superficiel 72e dans le tissu 7. On évite ainsi les problèmes associés à la pénétration du faisceau LASER dans le tissu 7. En effet, les bulles de gaz forment une barrière de bulles opaque (connue sous le nom de « OBL », sigle de l'expression anglaise « Opaque Bubble Layer ») empêchant la propagation de l'énergie issue du faisceau LASER sous celles-ci. Il est donc préférable de commencer par générer les bulles de gaz les plus profondes en priorité afin d'améliorer l'efficacité de l'appareil de découpe.

Avantageusement, l'utilisation d'un coupleur optique incluant une fibre optique 31 de type à cristal photonique à cœur creux (plutôt qu'un montage optique composé de miroirs afin de guider le faisceau LASER 11) permet de filtrer le signal LASER 11 issu du laser femtoseconde en supprimant ses éventuelles aberrations. Il est ainsi possible de réduire la distance entre deux plans de découpe successifs (distance entre plans de découpe successifs inférieure au diamètre d'un point d'impact) pour réaliser une découpe de grande précision dans un volume 74.

### 2.5. Unité de commande

Comme indiqué précédemment, l'unité de commande 6 permet de contrôler les différents éléments constituant l'appareil de découpe, à savoir le laser femtoseconde 1, le système de mise en forme 2, le scanner optique de balayage 4 et le système optique de focalisation 5.

L'unité de commande 6 est connectée à ces différents éléments par l'intermédiaire d'un (ou plusieurs) bus de communication permettant :
- la transmission de signaux de commande tels que
   - le masque de phase au système de mise en forme,
   - le signal d'activation au laser femtoseconde et les consignes de puissance,
   - la vitesse de balayage au scanner optique de balayage,
   - la position du scanner optique de balayage le long du chemin de déplacement,
   - la profondeur de découpe au système optique de focalisation.
- la réception de données de mesure issues des différents éléments du système tels que
   - la vitesse de balayage atteinte par le scanner optique, ou
   - la position du système optique de focalisation, etc.

L'unité de commande 6 peut être composée d'une (ou plusieurs) station(s) de travail, et/ou d'un (ou plusieurs) ordinateur(s) ou peut être de tout autre type connu de l'homme du métier. L'unité de commande 6 peut par exemple comprendre un téléphone portable, une tablette électronique (tel qu'un IPAD^{®}), un assistant personnel (ou « *PDA* », sigle de l'expression anglo-saxonne « *Personal Digital Assistant »),* etc. Dans tous les cas, l'unité de commande 6 comprend un processeur programmé pour permettre le pilotage du laser femtoseconde 1, du système de mise en forme 2, du scanner optique de balayage 4, du système optique de focalisation 5, etc.

### 2.6. Bras articulé

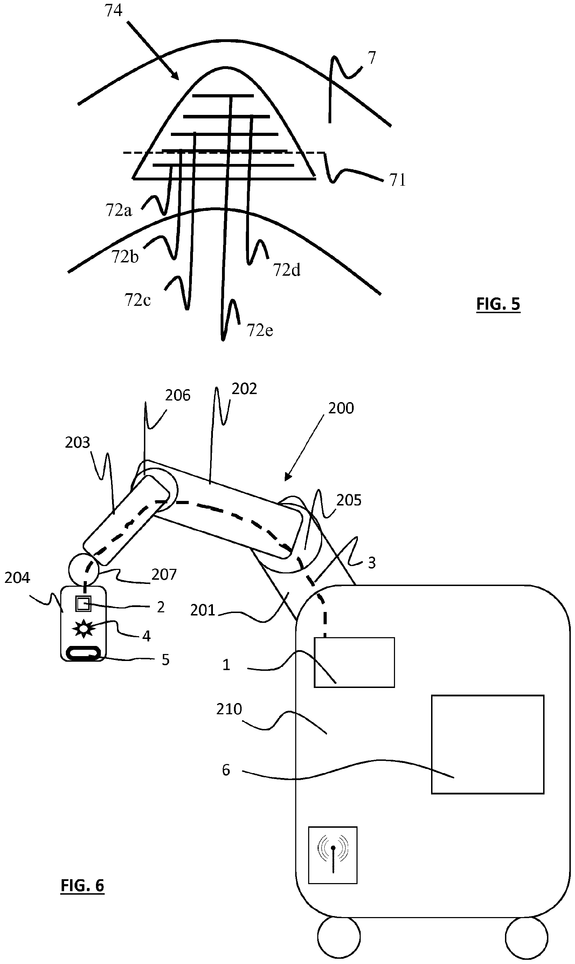

Grâce à l'utilisation d'un coupleur optique (3) incluant une fibre optique à cristal photonique (31), l'appareil de découpe décrit précédemment peut être monté dans un appareil de thérapie incluant un bras articulé 200 tel qu'illustré à la figure 6.

Le bras 200 comprend plusieurs segments de bras 201-204 reliés par des articulations 205-207 (liaisons pivot ou rotule) motorisées pour permettre le déplacement automatique en rotation des différents segments 201-204 les uns par rapport aux autres. En particulier, le bras est articulé pour permettre le déplacement de l'extrémité libre du bras selon trois axes orthogonaux X, Y et Z :
- l'axe X, définissant une direction longitudinale, horizontale,
- l'axe Y, définissant une direction transversale, horizontale, qui avec l'axe X définit un plan XY horizontal,
- l'axe Z, définissant une direction verticale, perpendiculaire au plan XY horizontal.

L'extrémité libre du bras 2 peut comporter un organe d'immobilisation équipé d'un anneau de succion capable de ventouser un tissu oculaire à traiter et de le maintenir fermement en position.

Le bras 2 est par exemple un TX260L commercialisé par la société STAUBLI. Avantageusement, le système de mise en forme 2, le scanner optique de balayage 4 et le système optique de focalisation 5 peuvent être montés dans le segment d'extrémité 204 du bras 200, tandis que le laser femtoseconde 1 peut être intégré à un caisson mobile 210 de l'appareil de thérapie, le coupleur optique 3 s'étendant entre le caisson 210 et le segment d'extrémité 204 pour propager le faisceau LASER 11 issu du laser femtoseconde 1 vers le système de mise en forme 2.

### 3. Conclusions

Ainsi, l'invention permet de disposer d'un outil de découpe efficace et précis. La modulation reconfigurable du front d'onde du faisceau LASER permet de générer de multiples points d'impact 81 simultanés ayant chacun une taille et une position contrôlée dans le plan de focalisation 71. Ces différents points d'impact 81 forment un motif 8 dans le plan focal 71 du faisceau LASER modulé.

L'utilisation d'un coupleur optique 3 incluant une fibre à cristal photonique 31 à cœur creux 311 permet de réduire la distance entre les différents points d'impacts formant le motif. En effet, en limitant le phénomène d'étalement du spectre lumineux, le coupleur optique incluant une fibre à cristal photonique à cœur creux permet de rendre le faisceau LASER modulé en phase plus propre.

L'invention est définie dans les revendications suivantes.

## Revendications

1. Appareil de découpe d'un tissu humain ou animal, tel qu'une cornée, ou un cristallin, ledit appareil incluant
- un laser femtoseconde (1) pour émettre un faisceau LASER initial sous la forme d'impulsions,
- un système de mise en forme (2) - tel qu'un modulateur spatial de lumière (SLM) - positionné en aval du laser femtoseconde (1), pour transformer le faisceau LASER initial en faisceau LASER modulé en phase, le système de mise en forme étant apte à moduler la phase du front d'onde du faisceau LASER initial selon une consigne de modulation calculée pour répartir l'énergie du faisceau LASER en au moins deux points d'impact (81) formant un motif (8) dans un plan de focalisation (71),
- un scanner optique de balayage (4), positionné en aval du système de mise en forme (2), pour déplacer le motif (8) le long d'un chemin de déplacement prédéfini dans le plan de focalisation (71),
- un système optique de focalisation (5), positionné en aval du scanner optique de balayage (4), pour déplacer le plan de focalisation (71) du faisceau LASER modulé dans un plan de découpe du tissu (7) désiré,
- une unité de commande (6) permettant de piloter le système de mise en forme (2), le scanner optique de balayage (4) et le système optique de focalisation (5),
***caractérisé en* ce que** l'appareil comprend en outre un coupleur optique (3) entre le laser femtoseconde (1) et le système de mise en forme (2), le coupleur optique (3) incluant :
- une fibre optique à cristal photonique (31) à cœur creux pour le filtrage du faisceau LASER (11) issu du laser femtoseconde (1), ladite fibre incluant un cœur creux (311), et au moins une gaine (312, 313) entourant le cœur creux (311),
- une première cellule de liaison (32) pour le raccordement du coupleur optique (3) au laser femtoseconde (1), et
- une deuxième cellule de liaison (33) pour le raccordement du coupleur optique (3) au système de mise en forme (2),
l'appareil comprenant en outre au moins une pompe à vide (P), chaque cellule de liaison (32, 33) comprenant au moins une borne de connexion (324, 334) débouchant vers l'extérieur de l'enveloppe (321, 331) et étant destinée à être raccordée à la pompe à vide (P).

2. Appareil de découpe selon la revendication 1, ***dans lequel*** chaque cellule de liaison (32, 33) est montée de manière étanche à une extrémité respective de la fibre à cristal photonique (31).

3. Appareil de découpe selon l'une quelconque des revendications 1 ou 2, ***dans lequel*** chaque cellule de liaison (32, 33) comprend :
- une enveloppe externe (321, 331),
- un canal de transmission (322, 332) logé dans l'enveloppe (321, 331), le canal de transmission (322, 332) permettant le passage du faisceau LASER (11) à l'intérieur de l'enveloppe (321, 331),
- un hublot (323, 333) transparent au rayonnement LASER à une extrémité du canal de transmission (322, 332), le hublot étant destiné à venir en regard du laser femtoseconde (1) ou du système de mise en forme (2).

4. Appareil de découpe selon la revendication 1, ***dans lequel*** l'unité de commande comprend des moyens aptes à piloter l'activation de la pompe à vide pour aspirer les gaz contenus dans le cœur creux de la fibre optique à cristal photonique (31).

## Patentansprüche

1. Vorrichtung zum Schneiden eines Gewebes eines Menschen oder eines Tieres wie eine Hornhaut oder eine Linse, wobei die Vorrichtung einschließt
- einen Femtosekundenlaser (1), um einen Ausgangslaserstrahl in Form von Impulsen zu senden,
- ein Formgebungssystem (2) - wie ein räumlicher Lichtmodulator (SLM) -, der nach dem Femtosekundenlaser (1) positioniert ist, um den Ausgangslaserstrahl in einen phasenmodulierten Laserstrahl umzuwandeln, wobei das Formgebungssystem imstande ist, die Phase der Wellenfront des Ausgangslaserstrahls gemäß einem Modulationssollwert zu modulieren, der berechnet ist, um die Energie des Laserstrahls auf mindestens zwei Auftreffpunkte (81) zu verteilen, die ein Motiv (8) in einer Fokusebene (71) bilden,
- einen optischen Abtastscanner (4), der nach dem Formgebungssystem (2) platziert ist, um das Motiv (8) entlang eines vorher festgelegten Verlagerungsweges in der Fokusebene (71) zu verlagern,
- ein optisches Fokussierungssystem (5), das nach dem optischen Abtastscanner (4) positioniert ist, um die Fokusebene (71) des modulierten Laserstrahls in einer gewünschten Schnittebene des Gewebes (7) zu verlagern,
- eine Steuereinheit (6), die erlaubt, das Formgebungssystem (2), den optischen Abtastscanner (4) und das optische Fokussierungssystem (5) zu steuern,
**dadurch gekennzeichnet, dass** die Vorrichtung ferner einen Optokoppler (3) zwischen dem Femtosekundenlaser (1) und dem Formgebungssystem (2) umfasst, wobei der Optokoppler (3) einschließt:
- eine optische Faser mit Photonenkristall (31) mit hohlem Kern für das Filtern des aus dem Femtosekundenlaser (1) ausgehenden Laserstrahls (11), wobei die Faser einen hohlen Kern (311) und mindestens einen Mantel (312, 313) einschließt, der den hohlen Kern (311) umgibt,
- eine erste Verbindungszelle (32) für den Anschluss des Optokopplers (3) an den Femtosekundenlaser (1), und
- eine zweite Verbindungszelle (33) für den Anschluss des Optokopplers (3) an das Formgebungssystem (2),
wobei die Vorrichtung ferner mindestens eine Vakuumpumpe (P) umfasst, wobei jede Verbindungszelle (32, 33) mindestens eine Anschlussklemme (324, 334) umfasst, die nach außerhalb des Mantels (321, 331) ausmündet und zum Anschluss an die Vakuumpumpe (P) bestimmt ist.

2. Schneidvorrichtung nach Anspruch 1, wobei jede Verbindungszelle (32, 33) dicht an einem jeweiligen Ende der Faser mit Photonenkristall (31) angebracht ist.

3. Schneidvorrichtung nach einem der Ansprüche 1 oder 2, wobei jede Verbindungszelle (32, 33) umfasst:
- einen äußeren Mantel (321, 331),
- einen Übertragungskanal (322, 332), der im Mantel (321, 331) untergebracht ist, wobei der Übertragungskanal (322, 332) den Durchgang des Laserstrahls (11) im Inneren des Mantels (321, 331) erlaubt,
- ein für die Laserstrahlung transparentes Fenster (323, 333) an einem Ende des Übertragungskanals (322, 332), wobei das Fenster bestimmt ist, dem Femtosekundenlaser (1) oder dem Formgebungssystem (2) zugewandt zu sein.

4. Schneidvorrichtung nach Anspruch 1, wobei die Steuereinheit Mittel umfasst, die imstande sind, die Aktivierung der Vakuumpumpe zu steuern, um die Gase anzusaugen, die im hohlen Kern der optischen Faser mit Photonenkristall (31) enthalten sind.

## Claims

1. An apparatus for cutting a human or animal tissue, such as a cornea or a crystalline lens, said apparatus including:
- a femtosecond laser (1) which emits an initial LASER beam in the form of pulses,
- a shaping system (2) - such as a Spatial Light Modulator (SLM) - positioned downstream of the femtosecond laser (1), to transform the initial LASER beam into a phase-modulated LASER beam, the shaping system being able to modulate the phase of the wave front of the initial LASER beam according to a modulation instruction calculated to distribute the energy of the LASER beam into at least two impact points (81) forming a pattern (8) in a focusing plane (71),
- an optical scanner (4), positioned downstream of the shaping system (2), to move the pattern (8) along a predefined movement path in the focusing plane (71),
- an optical focusing system (5), positioned downstream of the optical scanner (4), to move the focusing plane (71) of the modulated LASER beam in a desired cutting plane of the tissue(7),
- a control unit (6) that allows piloting the shaping system (2), the optical scanner (4) and the optical focusing system (5),
**Characterized in that** the apparatus further comprises an optical coupler (3) between the femtosecond laser (1) and the shaping system (2), the optical coupler (3) including:
- a hollow-core photonic-crystal optical fiber (31) for filtering the LASER beam (11) derived from the femtosecond laser (1), said fiber including a hollow core (311), and at least one sheath (312, 313) surrounding the hollow core (311),
- a first connection cell (32) for linking the optical coupler (3) to the femtosecond laser (1), and
- a second connection cell (33) for linking the optical coupler (3) to the shaping system (2),
the apparatus further comprising at least one vacuum pump (P), each connection cell (32, 33) comprising at least one connection terminal (324, 334) opening out towards the outside of the shell (321, 331) and being intended to be linked to the vacuum pump (P).

2. The cutting apparatus according to claim 1, wherein each connection cell (32, 33) is sealingly mounted at a respective end of the photonic-crystal fiber (31).

3. The cutting apparatus according to any one of claims 1 or 2, wherein each connection cell (32, 33) comprises:
- an outer shell (321, 331),
- a transmission channel (322, 332) housed in the shell (321, 331), the transmission channel (322, 332) allowing the passage of the LASER beam (11) inside the shell (321, 331),
- a window (323, 333) transparent to the LASER radiation at one end of the transmission channel (322, 332), the window being intended to face the femtosecond laser (1) or the shaping system (2).

4. The cutting apparatus according to claim 1, wherein the control unit comprises means able to pilot the activation of the vacuum pump to suck the gases contained in the hollow core of the photonic-crystal optical fiber (31).
